# FASCICULE DE BREVET EUROPEEN

(11) **EP 4 439 588 B1**
(45) Date de publication et mention de la délivrance du brevet: **25.02.2026**
(21) Numéro de dépôt: 24188992.2
(22) Date de dépôt: 14.12.2020
(51) Int. Cl.: G16H 10/60, G16H 20/10, G16H 80/00

(54) **SYSTÈME DE TITRATION À DISTANCE D'UN PATIENT ATTEINT DE LA MALADIE DE PARKINSON**
FERNTITRATIONSSYSTEM FÜR EINEN PATIENTEN MIT MORBUS PARKINSON
SYSTEM FOR REMOTE TITRATION OF PATIENT WITH PARKINSON'S DISEASE

(30) Priorité: 06.01.2020 FR 2000055
(43) Date de publication de la demande: 02.10.2024
(62) Demande divisionnaire de: 20213714.7
(73) Titulaire: Pharma Dom, 92220 Bagneux (FR); L'AIR LIQUIDE, SOCIETE ANONYME POUR L'ETUDE ET L'EXPLOITATION DES PROCEDES GEORGES CLAUDE, 75007 Paris (FR)
(72) Inventeur: KHUDYAKOV, Andrey, 92220 Bagneux (FR); DARNIS, Stéphanie, 92220 Bagneux (FR); RIQUIER, Véronique, 92220 Bagneux (FR)
(74) Mandataire: Air Liquide

(56) Documents cités:
- EP-A2- 2 410 448
- US-A1- 2008 086 086

## Description

L'invention concerne un système de titration, i.e. de recherche et modification de dose, à distance d'un patient atteint de la maladie de Parkinson, c'est-à-dire d'un patient parkinsonien, traité à son domicile par administration d'un médicament antiparkinsonien au moyen d'une pompe de perfusion, en particulier de l'apomorphine ou un mélange carbidopa/levodopa, ou toute autre molécule.

La maladie de Parkinson est une maladie neurologique chronique se caractérisant par une destruction des neurones à dopamine présentant dans le cerveau des individus atteints de cette maladie, appelés patients parkinsoniens.

Les neurones à dopamine sont impliqués dans le contrôle des mouvements du corps et leur destruction engendre alors différents symptômes chez ces patients parkinsoniens, comme des tremblements, des raideurs musculaires, une lenteur de mouvement, des mouvements involontaires plus ou moins importants, appelés dyskinésies, une rigidité musculaire, une instabilité posturale, des blocages (ou *Freezing Phase Off* en anglais)...

Souvent, l'un de ces symptômes est prépondérant chez un patient donné, c'est-à-dire qu'il s'agit du symptôme le plus gênant pour le patient considéré, donc devant être traité de façon prioritaire. On peut le qualifier de « symptôme prioritaire » ou de « critère d'efficacité ».

Pour traiter la maladie de Parkinson, on administre aux patients différents médicaments, notamment dopaminergiques, permettant de stimuler les neurones à dopamine.

Toutefois, il a été remarqué que ce type de médicament, en particulier la L-Dopa à administration orale, présente chez certains patients parkinsoniens des fluctuations d'efficacité.

Dès lors, chez les patients parkinsoniens présentant de telles fluctuations d'efficacité et/ou dans le cas de dyskinésies sévères, il est préconisé d'utiliser d'autres médicaments antiparkinsoniens sous forme liquide, telle l'apomorphine ou un mélange levodopa/carbidopa, qui sont des stimulateurs des récepteurs dopaminergiques postsynaptiques exerçant alors une action antiparkinsonienne.

Les médicaments antiparkinsoniens sous forme liquide, par exemple de type apomorphine, L-Dopa ou levodopa/carbidopa, sont généralement administrés au patient au moyen d'une pompe à perfusion délivrant des doses de médicament antiparkinsonien durant la journée et/ou la nuit au patient, via un cathéter implanté en sous-cutané et/ou comprenant la mise en place d'une sonde transabdominale externe ou d'une sonde intestinale interne chez le patient.

Il est indispensable, pour obtenir un traitement d'un patient parkinsonien pleinement efficace, de pouvoir délivrer au patient la bonne dose de médicament, en particulier de l'apomorphine, tout au long de la journée, c'est-à-dire la dose la plus efficace pour traiter (au moins) un symptôme prioritaire de la maladie de Parkinson lequel affecte ou gêne le plus le patient considéré.

Toutefois, chaque patient réagit de manière différente au traitement et le symptôme prioritaire à traiter en premier lieu est également différent d'un individu à un autre. Dès lors, pour déterminer la dose la plus efficace de médicament pour traiter un symptôme prioritaire chez un patient, le médecin doit opérer une titration du médicament antiparkinsonien qui est administré à ce patient, par exemple de l'apomorphine.

Or, les patients parkinsoniens ne sont généralement pas hospitalisés ou leur période d'hospitalisation est très courte et insuffisante pour définir correctement la dose la plus efficace vis-à-vis de leur(s) symptôme(s), étant donné que leur traitement a habituellement lieu à leur domicile, donc en l'absence de personnel soignant, i.e. médecin et/ou infirmière, pouvant réaliser la titration servant à déterminer la dose de médicament antiparkinsonien la plus efficace chez le patient considéré.

De plus, il est aussi souhaitable de pouvoir rectifier cette dose, c'est-à-dire la réduire ou l'augmenter, s'il est constaté que la dose ayant été fixée n'est pas ou pas assez efficace ou conduit à des effets secondaires négatifs chez le patient, tels que des allergies, des hallucinations, des troubles du comportement, des dyskinésies gênantes, une somnolence toute la journée, des nausées ou vomissements sous dompéridone...

EP-A-2410448 propose un système de pompe d'infusion avec une pompe commandée sur la base de réponses à des questions posées au patient chez qui la pompée est implantée.

Le problème est dès lors de pouvoir opérer une titration à distance (i.e. de recherche et modification de dose de médicament antiparkinsonien) d'un patient atteint de la maladie de Parkinson se trouvant hors hôpital, en particulier à son domicile, et suivant un traitement par perfusion d'un médicament contre la maladie de Parkinson, c'est-à-dire un médicament antiparkinsonien, telle que l'apomorphine, de la L-Dopa ou un mélange levodopa/carbidopa, telle la duodopa^{®}, ou analogue, qui lui injecté au moyen d'une pompe de perfusion de manière à pouvoir déterminer la dose la plus efficace de médicament antiparkinsonien chez le patient considéré vis-à-vis d'un symptôme prioritaire, typiquement le symptôme qui gêne le plus ce patient, et en prenant en compte d'éventuels effets indésirables susceptibles d'apparaître pendant la titration, et ce, afin de pouvoir rétroagir sur la pompe de perfusion et ainsi améliorer l'efficacité de traitement du patient considéré.

La solution de l'invention concerne un système de titration à distance d'un patient atteint de la maladie de Parkinson, c'est-à-dire un patient parkinsonien, traité hors hôpital, typiquement à son domicile, comprenant :
- un premier dispositif comprenant une première IHM (i.e. interface homme-machine) comprenant :
   - des premiers moyens de saisie permettant à un premier utilisateur, tel un personnel soignant, en particulier un médecin, de saisir des données de titration comprenant au moins :
      ▪ une pluralité de doses différentes d'un médicament antiparkinsonien à administrer à un patient au moyen d'une pompe de perfusion, et
      ▪ des durées d'administration journalières desdites doses de médicament antiparkinsonien audit patient au moyen d'une pompe de perfusion, et
   - des premiers moyens d'affichage configurés pour afficher au moins la pluralité de doses de médicament antiparkinsonien et les durées d'administration journalières saisies au moyen des premiers moyens de saisie, chaque dose de médicament antiparkinsonien étant affichée associée à une durée d'administration journalière,
- un second dispositif situé à distance du premier dispositif et comprenant une seconde IHM comprenant :
   - des seconds moyens de saisie permettant à un second utilisateur, tel un personnel soignant, par exemple un infirmier à domicile, de saisir des évaluations d'au moins un critère d'efficacité préfixé pour la pluralité de doses de médicament antiparkinsonien administrées au moyen de ladite pompe de perfusion, et
   - des seconds moyens d'affichage configurés pour afficher les évaluations saisies par le second utilisateur, chaque évaluation dudit au moins un critère d'efficacité étant affichée associée à une dose de médicament antiparkinsonien,
- des moyens de télécommunication configurés pour échanger des données entre les premier et second dispositifs,
- au moins un serveur informatique situé à distance desdits premier et second dispositifs, ledit serveur informatique :
   ▪ étant configuré pour traiter les données saisies au moyen des premier et second dispositifs de manière à déterminer la dose de médicament antiparkinsonien la plus efficace pour le patient considéré à partir de la pluralité de doses différentes de médicament antiparkinsonien provenant du premier dispositif et des évaluations provenant du second dispositif, et
   ▪ coopérant avec les moyens de télécommunication pour transmettre au premier dispositif au moins la dose de médicament antiparkinsonien la plus efficace pour le patient considéré ayant été déterminée,
et dans lequel les premiers et/ou les deuxièmes moyens d'affichage sont configurés pour afficher au moins la dose de médicament antiparkinsonien la plus efficace pour le patient considéré à administrer au moyen de ladite pompe de perfusion.

Selon le mode de réalisation considéré, le système de titration à distance de l'invention peut comprendre l'une ou plusieurs des caractéristiques suivantes :
- les premiers et/ou les deuxièmes moyens d'affichage sont configurés pour afficher au moins la dose de médicament antiparkinsonien la plus efficace pour le patient considéré fournie par les moyens de télécommunication.
- les premiers moyens de saisie permettent de saisir des doses de médicament antiparkinsonien comprises entre 0,1 et 12 mg/h, de préférence entre 0,5 et 5 mg/h.
- les doses de médicament antiparkinsonien sont exprimées en valeur de débit de la pompe d'administration servant à administrer/injecter ledit médicament, de préférence une valeur de débit comprise entre 0 et 8 ml/h ou entre 0 et 40 mg/h (pour une concentration de 5 mg/ml).
- les premiers moyens de saisie permettent de saisir des doses croissantes de médicament antiparkinsonien.
- les premiers moyens de saisie permettent de saisir des doses d'apomorphine, de L-Dopa ou de mélange levodopa/carbidopa, telle de la duodopa^{®}
- les premiers moyens de saisie sont en outre configurés pour permettre au premier utilisateur, tel un personnel soignant, par exemple un médecin neurologue, de sélectionner une dose maximale à ne pas dépasser chez le patient considéré.
- les premiers moyens de saisie permettent de saisir des durées d'administration journalières par saisie, pour chacune des doses de médicament antiparkinsonien :
   ▪ d'une heure de début d'administration et une heure de fin d'administration, ou
   ▪ d'une heure de début d'administration et un temps d'administration à partir de ladite heure de début d'administration.
- les premiers moyens de saisie permettent de saisir en outre un nombre de jours donné pour trouver une dose efficace pour un patient considéré.
- les seconds moyens de saisie permettent de saisir des évaluations d'au moins un critère d'efficacité préfixé pour la pluralité de doses de médicament antiparkinsonien sous forme d'une valeur numérique, en particulier exprimée en pourcentage (%), par exemple un pourcentage d'amélioration.
- les premiers moyens de saisie sont configurés pour permettre au premier utilisateur, tel un personnel soignant, en particulier un médecin neurologue, de sélectionner ou choisir le critère d'efficacité.
- les premiers moyens de saisie sont configurés pour permettre au premier utilisateur de sélectionner ou choisir un critère d'efficacité parmi plusieurs critères d'efficacité mémorisés ou prédéfinis.
- les premiers moyens de saisie sont configurés pour permettre au premier utilisateur de sélectionner ou entrer un niveau d'efficacité pour ledit critère d'efficacité, par exemple un pourcentage d'efficacité.
- les premiers moyens de saisie sont configurés pour permettre de modifier, ajuster ou adapter une ou des doses de médicament antiparkinsonien à administrer au patient, en particulier en fonction d'une apparition d'un ou plusieurs effets indésirables. En effet, pendant la phase de titration, pourront apparaître des effets indésirables, tels qu'allergies (hors nodules), confusion, hallucinations, troubles du comportement, dyskinésies gênantes, somnolence toute la journée, hypotension symptomatique, nausées/vomissement sous dompéridone ou autres, qui doivent être suivis et qui peuvent avoir un impact sur la phase de titration. Les doses de médicament, en particulier d'apomorphine, sont alors préférentiellement ajustées en fonction de leur apparition.
- les premiers moyens de saisie sont configurés pour permettre de saisir ou sélectionner au moins un effet indésirable, de préférence au sein d'une liste de plusieurs effets indésirables, par exemple une liste préenregistrée.
- les premiers moyens de saisie sont configurés pour permettre de saisir ou sélectionner au moins un effet indésirable et un degré de gêne dudit effet indésirable, par exemple « gênant » ou « peu gênant ».
- les premiers moyens de saisie sont configurés pour permettre de saisir ou sélectionner au moins un effet indésirable choisi parmi les allergies (hors nodules), la confusion, les hallucinations, les troubles du comportement, les dyskinésies gênantes, la somnolence toute la journée, l'hypotension symptomatique, les nausées ou vomissement sous dompéridone, ou autres.
- les premiers moyens d'affichage sont configurés pour afficher un ou plusieurs effets indésirables, de préférence une liste de plusieurs effets indésirables, par exemple une liste préenregistrée.
- les premiers moyens d'affichage sont configurés pour afficher un effet indésirable sélectionné parmi plusieurs effets indésirables et éventuellement un degré de gêne dudit effet indésirable
- les premiers moyens d'affichage sont configurés pour afficher une instruction d'adaptation du traitement en réponse à l'existence d'au moins un effet indésirable, de préférence l'instruction d'adaptation du traitement est choisie parmi une interruption du traitement, un maintien de la dose de médicament, une poursuite de la titration, une augmentation ou une diminution de la dose de médicament.
- les seconds moyens d'affichage sont configurés pour afficher une ou des informations comprenant la dose actuelle de médicament et éventuellement l'existence d'au moins un effet indésirable et éventuellement au moins une instruction d'adaptation du traitement.
- les seconds moyens de saisie sont configurés pour permettre de saisir ou sélectionner une nouvelle dose de médicament en fonction de l'existence d'au moins un effet indésirable et éventuellement d'au moins une instruction d'adaptation du traitement.
- les seconds moyens d'affichage sont configurés pour afficher ladite nouvelle dose de médicament saisie ou sélectionnée en fonction de l'existence d'au moins un effet indésirable et éventuellement d'au moins une instruction d'adaptation du traitement.
- selon un autre mode de réalisation de l'invention, le serveur informatique est configuré pour traiter les données saisies au moyen des premier et second dispositifs de manière à déterminer la dose de médicament antiparkinsonien la plus efficace pour le patient considéré à partir de la pluralité de doses différentes de médicament antiparkinsonien provenant du premier dispositif et des évaluations provenant du second dispositif, par rapport au niveau de dose efficace fixé par le personnel soignant.
- les seconds moyens de saisie permettent de saisir des évaluations d'au moins un critère d'efficacité préfixé chez le patient considéré choisi parmi les critères suivants :
   ▪ amélioration du blocage des mouvements (freezing),
   ▪ amélioration de la durée de blocage
   ▪ amélioration de la marche,
   ▪ diminution des tremblements,
   ▪ diminution des dyskinésies,
   ▪ diminution des fluctuations motrices,
   ▪ diminution des douleurs et
   ▪ amélioration de la qualité du sommeil,
   ▪ ou tout autre critère pouvant être fixé par un personnel soignant, tel un neurologue ou analogue.
- le critère d'efficacité est représentatif du traitement d'un symptôme prioritaire chez le patient considéré.
- l'évaluation du critère d'efficacité se fait par rapport à l'apparition desdits effets secondaires indésirables, par exemple du type allergie, confusion, hallucinations, troubles du comportement, dyskinésies gênantes, somnolence diurne, hypotension symptomatique, nausées ou vomissements sous dompéridone ou analogue, ....
- le premier dispositif et le second dispositif sont choisis parmi les ordinateurs portables ou fixes, les tablettes numériques et les téléphones intelligents (smartphones).
- les premiers moyens d'affichage comprennent un premier écran de visualisation et les seconds moyens d'affichage comprennent un second écran de visualisation.
- les premiers moyens de saisie comprennent un clavier numérique ou digital et les seconds moyens de saisie comprennent un clavier numérique ou digital.
- le serveur informatique comprend un ou plusieurs processeurs mettant en œuvre un ou plusieurs algorithmes, logiciels, programmes ou analogue.
- le serveur informatique comprend un module de communication, notamment de type BLE, WIFI, Carte à puce 2G/3G/4G/5G ou autre.
- les moyens d'affichage comprennent un ou des écrans de visualisation en couleurs ou monochrome (e.g. en noir et blanc).
- les moyens d'affichage comprennent un écran tactile.
- le serveur informatique est configuré pour déterminer la dose de médicament antiparkinsonien la plus efficace à partir d'une pluralité de doses différentes de médicament antiparkinsonien et d'évaluations d'au moins un critère d'efficacité préfixé pour la pluralité de doses de médicament antiparkinsonien.
- il comprend en outre une pompe de perfusion
- la pompe de perfusion est réglée ou commandée pour délivrer ladite dose de médicament antiparkinsonien la plus efficace pour le patient considéré.
- selon un mode de réalisation, la pompe de perfusion est commandée, de préférence à distance, pour délivrer la dose de médicament antiparkinsonien la plus efficace pour le patient considéré.
- le système est configuré pour opérer une injection du médicament antiparkinsonien, telle de l'apomorphine, automatiquement et en continu, de préférence pendant la journée, c'est-à-dire entre le lever et le coucher du patient P, ou inversement, durant la nuit, c'est-à-dire entre le coucher et le lever du patient P
- selon un autre mode de réalisation, la pompe de perfusion est réglée par le patient ou le personnel soignant à la dose de médicament antiparkinsonien la plus efficace pour le patient considéré.
- le système est configuré pour opérer une injection du médicament antiparkinsonien, telle de l'apomorphine, par bolus par activation de la pompe de perfusion par le patient.
- la pompe de perfusion comprend un réservoir contenant le médicament antiparkinsonien.
- le réservoir est un réservoir à piston.
- le réservoir est relié fluidiquement à un cathéter destiné à être implanté en sous-cutané chez le patient au moyen d'une aiguille d'injection sous-cutanée.
- le réservoir contient le médicament antiparkinsonien sous forme liquide.
- le réservoir a une contenance de 100 ml ou moins, par exemple de 50 ml ou moins.
- le réservoir est amovible.
- la pompe comprend une mémoire configurée pour mémoriser tout ou partie des paramètres de fonctionnement de la pompe, en particulier la dose (i.e. valeur de débit de perfusion) de médicament antiparkinsonien la plus efficace pour le patient considéré et/ou d'autres paramètres comme un horaire journalier de début de perfusion, un horaire journalier de fin de perfusion, un nombre maximum de bolus journaliers autorisés, un volume de bolus ou autres.
- la pompe comprend des moyens de pilotage, en particulier un (ou plusieurs) microprocesseur, configurés pour commander, directement ou indirectement, une fourniture (i.e. délivrance) de la dose (i.e. valeur de débit de perfusion) de médicament antiparkinsonien la plus efficace pour le patient considéré, en particulier de l'apomorphine.
- les moyens de pilotage sont configurés pour piloter un moteur électrique commandant un déplacement translatif d'un moyen d'actionnement, tel un poussoir ou une tige, agissant sur un piston du réservoir dans lequel est contenu le médicament antiparkinsonien, de sorte de repousser ledit piston en direction d'un orifice de distribution de fluide du réservoir et délivrer la dose désirée de médicament antiparkinsonien.
- la pompe est par ailleurs équipée de moyens de communication configurés pour recevoir des données provenant des premier et/ou second dispositifs, en particulier la dose de médicament antiparkinsonien la plus efficace utilisée pour modifier ou régler le débit de la pompe de perfusion. Tous les moyens de communication adaptés peuvent être utilisés, qu'ils utilisent des liaisons filaires ou sans fil, par exemple en mode wifi, Bluetooth^{®} ou autre.
- la pompe comprend une source de courant électrique, de préférence une pile ou une batterie, avantageusement rechargeable.

L'invention porte aussi sur une utilisation d'un système de titration selon l'invention pour réaliser une titration à distance d'un patient parkinsonien, c'est-à-dire d'un patient atteint de la maladie de Parkinson, traité à son domicile par administration d'un médicament antiparkinsonien au moyen d'une pompe de perfusion, en particulier apomorphine, carbidopa/levodopa, Duodopa^{®} ou toute autre molécule, de manière à déterminer la dose de médicament antiparkinsonien la plus efficace chez ce patient contre au moins un symptôme, en particulier un symptôme prioritaire, de ladite maladie de Parkinson tout en suivant préférentiellement l'apparition d'effets indésirables.

L'invention va maintenant être mieux comprise grâce à la description détaillée suivante, faite à titre illustratif mais non limitatif, en référence aux Figures annexées parmi lesquelles :
Fig. 1 schématise un patient parkinsonien traité par perfusion d'un médicament antiparkinsonien,
Fig. 2 schématise un mode de réalisation d'un système de titration à distance d'un patient parkinsonien selon l'invention,
Fig. 3 donne un exemple des informations affichées par le système de titration de l'invention, et
Fig. 4 à Fig. 8 donnent des exemples des informations affichées par le système de titration de l'invention en cas d'apparition ou d'existence d'un effet indésirable.

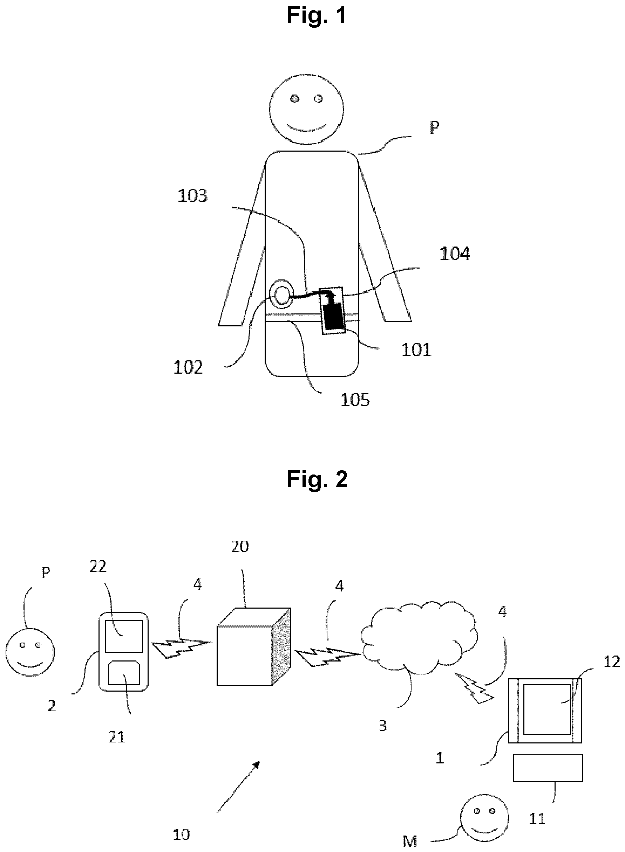

La Fig. 1 schématise une administration par perfusion d'un médicament antiparkinsonien stimulateur des récepteurs dopaminergiques, à savoir ici de l'apomorphine par exemple, chez un patient parkinsonien. Toutefois, un autre médicament peut être utilisé en lieu et place de l'apomorphine, comme de la L-Dopa ou un mélange carbidopa/levodopa (e.g. duodopa^{®}). On prend ci-après l'apomorphine comme exemple illustratif mais non limitatif.

Comme on le voit, l'administration de l'apomorphine (ou autre) se fait au moyen d'une pompe 101 de perfusion comprenant un réservoir 106 à piston relié à un cathéter 102 implanté en sous-cutané chez le patient P. Le réservoir 106 contenant l'apomorphine sous forme liquide a typiquement une contenant de quelques dizaines de ml, par exemple 20, 30, 50 ou 100 ml. Avantageusement, le réservoir 106 est amovible, c'est-à-dire qu'il peut être monté ou démonté du reste de la pompe 101, notamment pour le remplacer ou le remplir d'apomorphine lorsqu'il est vide.

Le cathéter 102 comprend une tubulure 103 raccordée à une sortie du réservoir de la pompe 101, servant à acheminer l'apomorphine jusqu'à une aiguille d'injection sous-cutanée qui permet de l'injecter au patient.

De préférence, un système adhésif ou analogue est prévu pour maintenir le cathéter en place sur le corps du patient. Il peut être fixé à différents endroits du corps, tels les flancs, les omoplates ou la zone péri-ombilicale.

Un tel ensemble de perfusion permet injecter l'apomorphine au patient P soit automatiquement et en continu pendant la journée, soit en bolus par activation de la pompe 101 par le patient lui-même, par exemple en cas de tremblement excessif nécessitant une délivrance d'une dose supplémentaire d'apomorphine.

En général, les injections d'apomorphine (ou autre médicament) se font pendant la journée, c'est-à-dire entre le lever et le coucher du patient P. Toutefois, dans certains cas, une ou des administrations d'apomorphine peuvent avoir lieu durant la nuit.

Les paramètres de fonctionnement de la pompe 101 sont réglés par un utilisateur, tel un médecin ou/et un infirmier, c'est-à-dire un professionnel de santé habilité, au niveau de la pompe et mémorisés au sein d'une mémoire intégrée à la pompe, en particulier une (ou des) valeur de débit de perfusion (i.e. dose de médicament), un horaire journalier de début de perfusion et un horaire journalier de fin de perfusion, et éventuellement un nombre maximum de bolus journaliers autorisés et un volume de bolus....

Par ailleurs, on prévoit aussi dans la pompe 101, des moyens de pilotage, tel un microprocesseur porté par une carte électronique, permettant de commander, directement ou indirectement, la fourniture d'apomorphine au débit désiré, par exemple en pilotant un moteur électrique commandant un déplacement translatif d'un moyen d'actionnement, tel un poussoir ou une tige, agissant sur le piston du réservoir dans lequel est contenue l'apomorphine de sorte de repousser ledit piston et donc aussi l'apomorphine (qui est poussée par le piston) en direction d'un orifice de distribution de fluide et alimentant ainsi en apomorphine la tubulure du cathéter 103.

La pompe 101 à apomorphine est préférentiellement insérée dans un étui 104, une pochette, un petit sac ou analogue que le patient P peut par exemple être fixée à sa ceinture 105 (comme illustré), portée en bandoulière ou accrochée autour du cou.

Bien entendu, la pompe de perfusion 101 peut aussi comprendre aussi d'autres composants habituels indispensables à son fonctionnement, comme une source de courant électrique, par exemple une pile ou batterie, de préférence rechargeable, un dispositif de mise en marche ou d'arrêt, par exemple un bouton ou une touche de type « on/off » (allumé/éteint), une ou plusieurs touches ou boutons de réglage ou de sélection ou autre, un écran d'affichage numérique...

La source de courant électrique alimente les différents composants de la pompe nécessitant du courant électrique pour fonctionner, en particulier moteur électrique, moyens de pilotage, notamment carte électronique et microprocesseur, mémoire...

La Fig. 2 schématise un mode de réalisation d'un système de titration 10 à distance, i.e. de choix de dose, selon l'invention d'un patient parkinsonien P, par exemple celui schématisé en Fig. 1, comprenant un premier dispositif 1 et un second dispositif 2 distants l'un de l'autre, et communicants 4 via un serveur informatique 3, notamment de type cloud ou nuage.

Le premier dispositif 1 comprend une première IHM 11, 12, c'est-à-dire une interface homme-machine, comprenant des premiers moyens de saisie 11, tel un clavier numérique ou mécanique, configurés pour permettre à un personnel soignant, typiquement un médecin neurologue M, de saisir des données de titration comprenant des doses différentes D1... Dn, de préférence croissantes, de médicament antiparkinsonien à administrer au patient P, telle de l'apomorphine, et des durées d'administration journalières desdites doses de médicament antiparkinsonien audit patient, par exemple une heure de début ou Td et une heure de fin ou Tf de traitement par perfusion via la pompe de perfusion 101.

Les doses croissantes D1... Dn d'apomorphine sont comprises par exemple entre 0 et 8 ml/h. Préférentiellement, les doses D sont exprimées en valeur de débit de perfusion d'apomorphine, typiquement compris entre 0,1 et 5 ml/h. Toutefois, elles peuvent être exprimées en une autre grandeur, par exemple en mg/h.

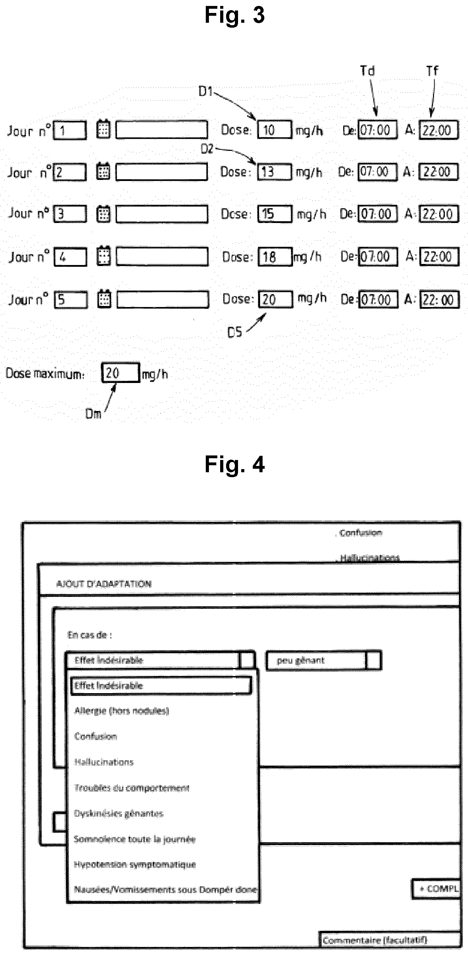

Les premiers moyens d'affichage 12, tel un écran d'affichage, sont configurés pour afficher la pluralité de doses D, par exemple D1 à D5, de médicament antiparkinsonien et les durées d'administration journalières (i.e. Td et Tf) ayant été saisies par le médecin M, chaque dose (e.g. D1 à D5) de médicament antiparkinsonien étant affichée associée à une durée d'administration journalière pour chaque jour considéré (e.g. Jour n°1 à Jour n°5), par exemple côté à côte sur l'écran d'affichage/visualisation des premiers moyens d'affichage 12, comme illustré en Fig. 3.

Par exemple, les doses D1-D5 données en Fig. 3 sont des débits de perfusion d'apomorphine de 1, 1.3, 1.5, 1.8 et 2 ml/h, dont le début d'administration est fixé à 07:00 (Td) et la fin à 22:00 de chaque jour (J1-J5).

Les premiers moyens d'affichage 12 sont en outre configurés pour afficher une dose maximale (Dm) ayant été choisie par le médecin M, à savoir ici 2 ml/h. Bien entendu, une dose supérieure ou inférieure pourrait être choisie.

Le critère d'efficacité est généralement choisi par le médecin M après discussion avec le patient P. Le critère d'efficacité préfixé considéré peut être par exemple choisi parmi les critères suivants : amélioration du blocage des mouvements (i.e. freezing), amélioration de la durée de blocage, amélioration de la marche, diminution des tremblements, diminution des dyskinésies, diminution des fluctuations motrices, diminution des douleurs et amélioration de la qualité du sommeil.

De là, les premiers moyens de saisie 11 sont aussi configurés pour permettre au médecin M, de sélectionner un critère d'efficacité et de sélectionner ou entrer un niveau de dose efficace pour ledit critère d'efficacité.

Par ailleurs, le second dispositif 2 est situé à distance du premier dispositif 1, typiquement à une distance de plusieurs centaines de mètres, voire à plusieurs kilomètres ou dizaines de kilomètres. Il comprenant une seconde IHM 21, 22 comprenant elle aussi des seconds moyens de saisie 21 et des seconds moyens d'affichage 22, tel un écran d'affichage.

Les seconds moyens de saisie 21 sont configurés pour permettre à un patient (P) de saisir des évaluations d'un (ou des) critère d'efficacité préfixé pour la pluralité de doses D de médicament antiparkinsonien, et les seconds moyens d'affichage 22 sont quant à eux configurés pour afficher les évaluations saisies par le patient P, chaque évaluation du critère d'efficacité, par exemple un % d'amélioration, étant affichée associée à une dose de médicament antiparkinsonien.

De préférence, le premier dispositif 1 et le second dispositif 2 sont choisis parmi les ordinateurs, les tablettes numériques et les téléphones intelligents (smartphone). Par exemple, le premier dispositif 1 est un ordinateur et le second dispositif 2 est un téléphone intelligent, comme illustré en Fig. 2.

Par ailleurs, les échanges d'informations et de données entre les premier et second dispositifs 1, 2 et le serveur informatique 3 se font via des moyens de télécommunication 4, par exemple des moyens émetteurs/récepteurs et un (ou des) réseau de télécommunication, tel le réseau GSM.

De préférence, les moyens de transmission de signal 4 sont configurés pour transmettre à distance via le réseau internet ou un autre réseau de communication, tels les réseaux GSM, Wifi, Lora ou Sigfox. Par exemple, ils comprennent un modem de type GSM 20 ou autre et une antenne émettrice.

Selon l'invention, le serveur informatique 3 est configuré pour traiter les données saisies au moyen des premier et second dispositifs 1, 2 de manière à déterminer la dose de médicament antiparkinsonien la plus efficace pour le patient considéré à partir de la pluralité de doses différentes de médicament antiparkinsonien provenant du premier dispositif 1 et des évaluations provenant du second dispositif 2 et coopère avec les moyens de télécommunication 4 pour transmettre au premier dispositif 1 la dose de médicament antiparkinsonien la plus efficace pour le patient (P) considéré ayant été déterminée.

La dose de médicament antiparkinsonien la plus efficace proposée peut ensuite être affichée par les premiers moyens d'affichage 12, voire valider ensuite par le médecin M.

Par ailleurs, comme illustré sur Fig. 4 à Fig. 8 le système de titration de l'invention peut aussi permettre d'afficher des informations spécifiques en cas d'existence ou d'apparition d'un (ou plusieurs) effet indésirable chez le patient.

Ainsi, les premiers moyens de saisie 11 sont aussi configurés pour permettre au premier utilisateur, tel un neurologue, de modifier, ajuster ou adapter une ou des doses de médicament antiparkinsonien à administrer au patient, par exemple de l'apomorphine, en fonction de l'apparition d'un ou plusieurs effets indésirables. En effet, pendant la phase de titration, peuvent apparaître des effets indésirables chez le patient, tels qu'allergies (hors nodules), confusion, hallucinations, troubles du comportement, dyskinésies gênantes, somnolence toute la journée, hypotension symptomatique, nausées/vomissement sous dompéridone ou autres, qui doivent être suivis et qui peuvent avoir un impact sur la phase de titration. Les doses de médicament, en particulier d'apomorphine, sont alors préférentiellement ajustées en fonction de leur apparition.

Pour ce faire, comme illustré en Fig. 4, les premiers moyens d'affichage 12 sont alors configurés pour afficher plusieurs effets indésirables sélectionnables au sein d'une liste de plusieurs effets indésirables, par exemple une liste préenregistrée, à savoir ici un menu déroulant, et les premiers moyens de saisie 11 sont configurés pour permettre de saisir au moins un desdits effets indésirables.

Avantageusement, l'effet indésirable peut être choisi dans une liste, tel un menu déroulant, comprenant les allergies (hors nodules), la confusion, les hallucinations, les troubles du comportement, les dyskinésies gênantes, la somnolence toute la journée, l'hypotension symptomatique ou les nausées ou vomissement sous dompéridone.

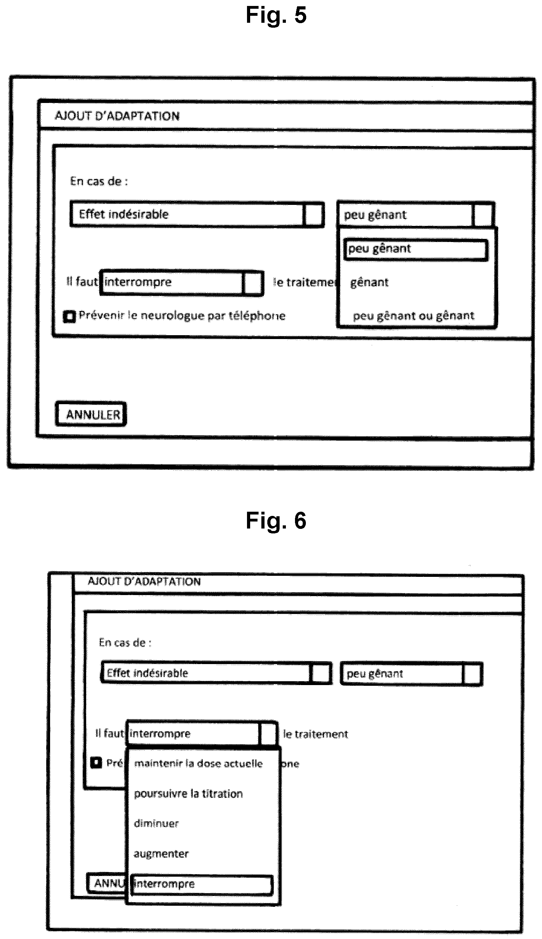

Ensuite, les premiers moyens de saisie 11 permettent de saisir ou sélectionner un degré de gêne (i.e. de sévérité) lié à cet effet indésirable, par exemple « gênant » ou « peu gênant », et les premiers moyens d'affichage 12 permettent de visualiser ce choix, comme illustré en Fig. 5.

Comme schématisé en Fig. 6, les premiers moyens d'affichage 12 sont alors configurés pour afficher une instruction d'adaptation du traitement en réponse à l'existence d'au moins un effet indésirable, de préférence l'instruction d'adaptation du traitement est choisie parmi une interruption du traitement, un maintien de la dose de médicament, une poursuite de la titration, une augmentation ou une diminution de la dose de médicament.

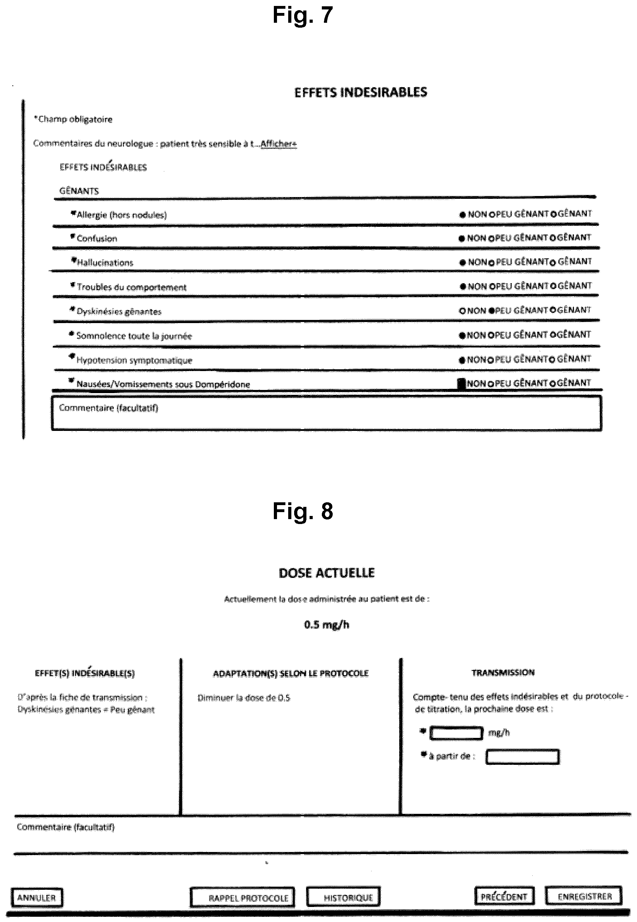

Avantageusement, les premiers moyens d'affichage 12 peuvent afficher un récapitulatif à l'attention du premier utilisateur, tel un neurologue, du ou des effets indésirables et de leur degré de gêne ou de sévérité chez le patient considéré, comme schématisé en Fig. 7, de manière à lui permettre de se faire une opinion et éventuellement d'adapter les doses de médicament.

Comme illustré en Fig. 8, les premiers moyens d'affichage 12 aussi sont configurés pour afficher une ou des informations comprenant la dose actuelle de médicament, par exemple exprimée en mg/h (e.g. ici 5 mg/h), et éventuellement l'existence d'au moins un effet indésirable et au moins une instruction d'adaptation du traitement, et les premiers moyens de saisie 11 sont, quant à eux, configurés pour permettre de saisir ou sélectionner une nouvelle dose de médicament en fonction de l'existence de l'effet indésirable et de l'instruction d'adaptation du traitement s'affichant sur l'écran des premiers moyens d'affichage 12, et d'une date à partir de laquelle appliquer cette nouvelle dose.

D'une façon générale, le second dispositif 2 permet aussi d'opérer une collecte d'autres informations ou données, à savoir non seulement le ou les paramètres de fonctionnement provenant de la pompe 101, tel le débit de perfusion, mais aussi d'autres informations, données cliniques, paramètres ou autres provenant d'un (ou plusieurs) autre dispositif médical connecté et/ou des réponses à des questionnaires de santé ou autre, puis leur analyse et une restitution des données vers un ou des utilisateurs ou intervenants, tel le personnel soignant, notamment médecin, infirmier/ère ou autre à l'hôpital ou à domicile.

Dans tous les cas, une fois que la dose de médicament antiparkinsonien la plus efficace pour le patient considéré a été déterminée, la pompe de perfusion 101 peut être réglée ou commandée, de préférence à distance, pour délivrer ladite dose de médicament antiparkinsonien audit patient.

Comme expliqué ci-avant, ceci se fait par commande de la pompe de perfusion 101 par des moyens de pilotage à microprocesseur qui agissent sur le piston de la pompe de perfusion 101 afin de délivrer le débit de médicament antiparkinsonien correspondant à la dose de médicament antiparkinsonien la plus efficace pour le patient considéré.

Les moyens de pilotage sont préférentiellement embarqués dans la pompe de perfusion 101.

Avantageusement, la pompe est également équipée de moyens de communication configurés pour recevoir des données provenant des premier et/ou second dispositifs, en particulier la dose de médicament antiparkinsonien la plus efficace pour le patient considéré, laquelle est utilisée pour modifier ou régler le débit de la pompe de perfusion.

Le système de titration de l'invention permet donc de rechercher et modifier ou ajuster, et/ou valider, de préférence à distance, la dose de médicament la plus efficace à injecter à un patient de manière à lui administrer un traitement qui soit le plus efficace possible, en particulier du fait d'une prise en compte d'un ou plusieurs critères d'efficacité chez le patient considéré.

## Revendications

1. Système de titration à distance (10) d'un patient (P) atteint de la maladie de Parkinson comprenant :
- un premier dispositif (1) comprenant une première IHM (11, 12) comprenant :
• des premiers moyens de saisie (11) permettant à un premier utilisateur, en particulier un médecin neurologue (M), de saisir des données de titration comprenant au moins :
- une pluralité de doses différentes d'un médicament antiparkinsonien à administrer à un patient au moyen d'une pompe (101) de perfusion, lesdites doses étant comprises entre 0,1 et 12 mg/h, et
- des durées d'administration journalières desdites doses de médicament antiparkinsonien audit patient au moyen de ladite pompe (101) de perfusion, lesdites durées d'administration journalières comprennent : une heure de début d'administration et une heure de fin d'administration, ou une heure de début d'administration et un temps d'administration à partir de ladite heure de début d'administration, et
• des premiers moyens d'affichage (12) configurés pour afficher au moins la pluralité de doses de médicament antiparkinsonien et les durées d'administration journalières saisies au moyen des premiers moyens de saisie (11), chaque dose de médicament antiparkinsonien étant affichée associée à une durée d'administration journalière,
- un second dispositif (2) situé à distance du premier dispositif (1) et comprenant une seconde IHM (21, 22) comprenant :
• des seconds moyens de saisie (21) permettant à un second utilisateur de saisir des évaluations d'au moins un critère d'efficacité préfixé pour la pluralité de doses de médicament antiparkinsonien administrées au moyen de ladite pompe (101) de perfusion,
• et des seconds moyens d'affichage (22) configurés pour afficher les évaluations saisies par le second utilisateur, chaque évaluation dudit au moins un critère d'efficacité étant affichée associée à une dose de médicament antiparkinsonien,
- des moyens de télécommunication (4) configurés pour échanger des données entre les premier et second dispositifs (1, 2), et
- au moins un serveur informatique (3) situé à distance desdits premier et second dispositifs (1, 2), ledit serveur informatique (3) :
▪ étant configuré pour traiter les données saisies au moyen des premier et second dispositifs (1, 2) de manière à déterminer la dose de médicament antiparkinsonien la plus efficace pour le patient considéré à partir de la pluralité de doses différentes de médicament antiparkinsonien provenant du premier dispositif (1) et des évaluations provenant du second dispositif (2), et
▪ coopérant avec les moyens de télécommunication (4) pour transmettre au premier dispositif (1) au moins la dose de médicament antiparkinsonien la plus efficace pour le patient (P) considéré ayant été déterminée,
et dans lequel les premiers et/ou les deuxièmes moyens d'affichage (12) sont configurés pour afficher au moins la dose de médicament antiparkinsonien la plus efficace pour le patient considéré à administrer au moyen de ladite pompe (101) de perfusion.

2. Système de titration selon la revendication précédente, **caractérisé en ce qu'**il comprend en outre une pompe de perfusion (101) réglée ou commandée, de préférence à distance, pour délivrer ladite dose de médicament antiparkinsonien la plus efficace pour le patient considéré.

3. Système de titration selon l'une des revendications précédentes, **caractérisé en ce que** les premiers moyens de saisie (11) permettent de saisir des doses de médicament antiparkinsonien comprises entre 0,5 et 5 mg/h.

4. Système de titration selon l'une des revendications précédentes, **caractérisé en ce que** les premiers moyens de saisie (11) permettent de saisir des doses croissantes de médicament antiparkinsonien.

5. Système de titration selon l'une des revendications précédentes, **caractérisé en ce que** les premiers moyens de saisie (11) permettent de saisir des doses d'apomorphine, de L-Dopa ou de levodopa/carbidopa, en particulier d'apomorphine.

6. Système de titration selon l'une des revendications précédentes, **caractérisé en ce que** les seconds moyens de saisie (21) permettent à un second utilisateur, de préférence un patient (P), de saisir des évaluations d'au moins un critère d'efficacité préfixé pour la pluralité de doses de médicament antiparkinsonien sous forme d'une valeur numérique, en particulier exprimée en pourcentage (%).

7. Système selon la revendication précédente, **caractérisé en ce que** les seconds moyens de saisie (21) permettent de saisir des évaluations d'au moins un critère d'efficacité préfixé chez le patient (P) considéré choisi parmi les critères suivants : amélioration du blocage des mouvements (freezing), amélioration de la durée de blocage, amélioration de la marche, diminution des tremblements, diminution des dyskinésies, diminution des fluctuations motrices, diminution des douleurs et amélioration de la qualité du sommeil.

8. Système selon la revendication 1, **caractérisé en ce que** le premier dispositif (1) et le second dispositif (2) sont choisis parmi les ordinateurs portables ou fixes, les tablettes numériques et les téléphones intelligents.

9. Système selon l'une des revendications précédentes, **caractérisé en ce que** les premiers moyens de saisie (11) sont configurés pour permettre au premier utilisateur (M), de préférence un médecin neurologue, de sélectionner le critère d'efficacité et de sélectionner ou entrer un niveau de dose efficace pour ledit critère d'efficacité.

10. Système selon l'une des revendications précédentes, **caractérisé en ce que** :
- les premiers moyens de saisie (11) sont configurés pour permettre de saisir ou sélectionner au moins un effet indésirable et un degré de gêne dudit effet indésirable, et
- les premiers moyens d'affichage (12) sont configurés pour permettre de visualiser ledit au moins un effet indésirable et ledit degré de gêne dudit effet indésirable.

11. Système selon l'une des revendications précédentes, **caractérisé en ce qu'**il comprend en outre des moyens pour permettre de valider la dose de médicament antiparkinsonien la plus efficace ayant été déterminée.

12. Système selon l'une des revendications précédentes, **caractérisé en ce que** les premiers moyens d'affichage (12) sont en outre configurés pour afficher une instruction d'adaptation du traitement en réponse à l'existence d'au moins un effet indésirable, l'instruction d'adaptation du traitement étant choisie parmi une interruption du traitement, un maintien de la dose de médicament, une poursuite de la titration, une augmentation ou une diminution de la dose de médicament.

13. Système selon l'une des revendications précédentes, **caractérisé en ce que** la pompe (101) de perfusion comprend un réservoir contenant le médicament antiparkinsonien et des moyens de pilotage configurés pour commander la fourniture de la dose de médicament antiparkinsonien la plus efficace pour le patient considéré.

14. Système selon la revendication 1, **caractérisé en ce que** les premiers moyens de saisie (11) sont en outre configurés pour permettre de saisir ou sélectionner une nouvelle dose de médicament en fonction de l'existence de l'effet indésirable et de l'instruction d'adaptation du traitement s'affichant sur l'écran des premiers moyens d'affichage (12), et d'une date à partir de laquelle appliquer cette nouvelle dose.

15. Système selon la revendication 1, **caractérisé en ce que** le serveur informatique (3) est configuré pour déterminer la dose de médicament antiparkinsonien la plus efficace à partir d'une pluralité de doses différentes de médicament antiparkinsonien et d'évaluations d'au moins un critère d'efficacité préfixé pour la pluralité de doses de médicament antiparkinsonien.

## Patentansprüche

1. Fern-Titrationssystem (10) für einen Patienten (P), der an der Parkinson-Krankheit leidet, umfassend:
- eine erste Vorrichtung (1), die eine erste HMI (11, 12) umfasst, die Folgendes umfasst:
• erste Eingabemittel (11), die es einem ersten Benutzer, insbesondere einem Neurologen (M), ermöglichen, Titrationsdaten einzugeben, die mindestens Folgendes umfassen:
- eine Vielzahl verschiedener Dosen eines Antiparkinson-Medikaments, das einem Patienten mittels einer Infusionspumpe (101) zu verabreichen ist, wobei die Dosen zwischen 0,1 und 12 mg/h liegen, und
- tägliche Verabreichungsdauern der Dosen des Antiparkinson-Medikaments an den Patienten mittels der Infusionspumpe (101), wobei die täglichen Verabreichungsdauern Folgendes umfassen: eine Startzeit der Verabreichung und eine Endzeit der Verabreichung, oder eine Startzeit der Verabreichung und eine Verabreichungsdauer ab der Startzeit der Verabreichung, und
• erste Anzeigemittel (12), die konfiguriert sind, um mindestens die Vielzahl von Dosen des Antiparkinson-Medikaments und die täglichen Verabreichungsdauern anzuzeigen, die mittels der ersten Eingabemittel (11) eingegeben wurden, wobei jede Dosis des Antiparkinson-Medikaments in Verbindung mit einer täglichen Verabreichungsdauer angezeigt wird,
- eine zweite Vorrichtung (2), die sich in einem Abstand von der ersten Vorrichtung (1) befindet und eine zweite HMI (21, 22) umfasst, die Folgendes umfasst:
• zweite Eingabemittel (21), die es einem zweiten Benutzer ermöglichen, Bewertungen von mindestens einem vordefinierten Wirksamkeitskriterium für die Vielzahl von Dosen des Antiparkinson-Medikaments einzugeben, die mittels der Infusionspumpe (101) verabreicht werden,
• und zweite Anzeigemittel (22), die konfiguriert sind, um die vom zweiten Benutzer eingegebenen Bewertungen anzuzeigen, wobei jede Bewertung des mindestens einen Wirksamkeitskriteriums in Verbindung mit einer Dosis des Antiparkinson-Medikaments angezeigt wird,
- Telekommunikationsmittel (4), die konfiguriert sind, um Daten zwischen der ersten und der zweiten Vorrichtung (1, 2) auszutauschen, und
- mindestens einen Computerserver (3), der sich in einem Abstand von der ersten und zweiten Vorrichtung (1, 2) befindet, wobei der Computerserver (3):
%^{a} **konfiguriert ist, um die mittels d** Vorrichtung (1, 2) eingegebenen Daten zu verarbeiten, um die wirksamste Dosis des Antiparkinson-Medikaments für den betreffenden Patienten aus der Vielzahl verschiedener Dosen des Antiparkinson-Medikaments, die von der ersten Vorrichtung (1) stammen, und den Bewertungen, die von der zweiten Vorrichtung (2) stammen, zu bestimmen, und
%^{a} **mit den Telekommunikationsmitteln** die erste Vorrichtung (1) mindestens die wirksamste Dosis des Antiparkinson-Medikaments für den betreffenden Patienten (P) zu übertragen, die bestimmt wurde,
- und wobei die ersten und/oder zweiten Anzeigemittel (12) konfiguriert sind, um mindestens die wirksamste Dosis des Antiparkinson-Medikaments für den betreffenden Patienten anzuzeigen, die mittels der Infusionspumpe (101) zu verabreichen ist.

2. Titrationssystem nach dem vorhergehenden Anspruch, **dadurch gekennzeichnet, dass** es ferner eine Infusionspumpe (101) umfasst, die, vorzugsweise aus der Ferne, eingestellt oder gesteuert wird, um die wirksamste Dosis des Antiparkinson-Medikaments für den betreffenden Patienten zu verabreichen.

3. Titrationssystem nach einem der vorhergehenden Ansprüche, **dadurch gekennzeichnet, dass** die ersten Eingabemittel (11) die Eingabe von Dosen des Antiparkinson-Medikaments zwischen 0,5 und 5 mg/h ermöglichen.

4. Titrationssystem nach einem der vorhergehenden Ansprüche, **dadurch gekennzeichnet, dass** die ersten Eingabemittel (11) die Eingabe steigender Dosen des Antiparkinson-Medikaments ermöglichen.

5. Titrationssystem nach einem der vorhergehenden Ansprüche, **dadurch gekennzeichnet, dass** die ersten Eingabemittel (11) die Eingabe von Dosen von Apomorphin, L-Dopa oder Levodopa/Carbidopa, insbesondere Apomorphin, ermöglichen.

6. Titrationssystem nach einem der vorhergehenden Ansprüche, **dadurch gekennzeichnet, dass** die zweiten Eingabemittel (21) es einem zweiten Benutzer, vorzugsweise einem Patienten (P), ermöglichen, Bewertungen von mindestens einem vordefinierten Wirksamkeitskriterium für die Vielzahl von Dosen des Antiparkinson-Medikaments in Form eines numerischen Werts, insbesondere als Prozentsatz (%) ausgedrückt, einzugeben.

7. System nach dem vorhergehenden Anspruch, **dadurch gekennzeichnet, dass** die zweiten Eingabemittel (21) die Eingabe von Bewertungen von mindestens einem vordefinierten Wirksamkeitskriterium beim betreffenden Patienten (P) ermöglichen, das aus den folgenden Kriterien ausgewählt ist:
Verbesserung der Bewegungsblockade (Freezing), Verbesserung der Blockadedauer, Verbesserung des Gangs, Verringerung des Zitterns, Verringerung der Dyskinesien, Verringerung der motorischen Fluktuationen, Verringerung der Schmerzen und Verbesserung der Schlafqualität.

8. System nach Anspruch 1, **dadurch gekennzeichnet, dass** die erste Vorrichtung (1) und die zweite Vorrichtung (2) aus tragbaren oder stationären Computern, digitalen Tablets und intelligenten Telefonen ausgewählt sind.

9. System nach einem der vorhergehenden Ansprüche, **dadurch gekennzeichnet, dass** die ersten Eingabemittel (11) konfiguriert sind, um es dem ersten Benutzer (M), vorzugsweise einem Neurologen, zu ermöglichen, das Wirksamkeitskriterium auszuwählen und eine wirksame Dosisstufe für das Wirksamkeitskriterium auszuwählen oder einzugeben.

10. System nach einem der vorhergehenden Ansprüche, **dadurch gekennzeichnet, dass**:
- die ersten Eingabemittel (11) konfiguriert sind, um die Eingabe oder Auswahl von mindestens einer unerwünschten Wirkung und einem Grad der Beeinträchtigung durch die unerwünschte Wirkung zu ermöglichen, und
- die ersten Anzeigemittel (12) konfiguriert sind, um die Visualisierung der mindestens einen unerwünschten Wirkung und des Grades der Beeinträchtigung durch die unerwünschte Wirkung zu ermöglichen.

11. System nach einem der vorhergehenden Ansprüche, **dadurch gekennzeichnet, dass** es ferner Mittel umfasst, um die Validierung der wirksamsten Dosis des Antiparkinson-Medikaments zu ermöglichen, die bestimmt wurde.

12. System nach einem der vorhergehenden Ansprüche, **dadurch gekennzeichnet, dass** die ersten Anzeigemittel (12) ferner konfiguriert sind, um eine Anweisung zur Behandlungsanpassung als Reaktion auf das Vorhandensein von mindestens einer unerwünschten Wirkung anzuzeigen, wobei die Anweisung zur Behandlungsanpassung aus einer Unterbrechung der Behandlung, einer Beibehaltung der Medikamentendosis, einer Fortsetzung der Titration, einer Erhöhung oder einer Verringerung der Medikamentendosis ausgewählt wird.

13. System nach einem der vorhergehenden Ansprüche, **dadurch gekennzeichnet, dass** die Infusionspumpe (101) ein Reservoir, das das Antiparkinson-Medikament enthält, und Steuermittel umfasst, die konfiguriert sind, um die Zufuhr der wirksamsten Dosis des Antiparkinson-Medikaments für den betreffenden Patienten zu steuern.

14. System nach Anspruch 1, **dadurch gekennzeichnet, dass** die ersten Eingabemittel (11) ferner konfiguriert sind, um die Eingabe oder Auswahl einer neuen Medikamentendosis in Abhängigkeit von dem Vorhandensein der unerwünschten Wirkung und der auf dem Bildschirm der ersten Anzeigemittel (12) angezeigten Anweisung zur Behandlungsanpassung sowie eines Datums, ab dem diese neue Dosis anzuwenden ist, zu ermöglichen.

15. System nach Anspruch 1, **dadurch gekennzeichnet, dass** der Computerserver (3) konfiguriert ist, um die wirksamste Dosis des Antiparkinson-Medikaments aus einer Vielzahl verschiedener Dosen des Antiparkinson-Medikaments und Bewertungen von mindestens einem vordefinierten Wirksamkeitskriterium für die Vielzahl von Dosen des Antiparkinson-Medikaments zu bestimmen.

## Claims

1. A remote titration system (10) for a patient (P) suffering from Parkinson's disease, comprising:
- a first device (1) comprising a first HMI (11, 12) comprising:
• first input means (11) for allowing a first user, in particular a neurologist (M), to enter titration data comprising at least:
- a plurality of different doses of an antiparkinsonian drug to be administered to a patient by means of an infusion pump (101), said doses being between 0.1 and 12 mg/h, and
- daily administration durations for said doses of antiparkinsonian drug to said patient by means of said infusion pump (101), said daily administration durations comprising: an administration start time and an administration end time, or an administration start time and an administration duration from said administration start time, and
• first display means (12) configured to display at least the plurality of doses of antiparkinsonian drug and the daily administration durations entered by means of the first input means (11), each dose of antiparkinsonian drug being displayed associated with a daily administration duration,
- a second device (2) located at a distance from the first device (1) and comprising a second HMI (21, 22) comprising:
• second input means (21) for allowing a second user to enter evaluations of at least one predefined efficacy criterion for the plurality of doses of antiparkinsonian drug administered by means of said infusion pump (101),
• and second display means (22) configured to display the evaluations entered by the second user, each evaluation of said at least one efficacy criterion being displayed associated with a dose of antiparkinsonian drug,
- telecommunication means (4) configured to exchange data between the first and second devices (1, 2), and
- at least one computer server (3) located at a distance from said first and second devices (1, 2), said computer server (3):
%^{a} **being configured to process the da** first and second devices (1, 2) so as to determine the most effective dose of antiparkinsonian drug for the patient concerned from the plurality of different doses of antiparkinsonian drug originating from the first device (1) and the evaluations originating from the second device (2), and
%^{a} **cooperating with the telecommunica** to the first device (1) at least the most effective dose of antiparkinsonian drug for the patient (P) concerned that has been determined,
- and wherein the first and/or second display means (12) are configured to display at least the most effective dose of antiparkinsonian drug for the patient concerned to be administered by means of said infusion pump (101).

2. The titration system according to the preceding claim, **characterized in that** it further comprises an infusion pump (101) that is adjusted or controlled, preferably remotely, to deliver said most effective dose of antiparkinsonian drug for the patient concerned.

3. The titration system according to any one of the preceding claims, **characterized in that** the first input means (11) allow for the entry of doses of antiparkinsonian drug between 0.5 and 5 mg/h.

4. The titration system according to any one of the preceding claims, **characterized in that** the first input means (11) allow for the entry of increasing doses of antiparkinsonian drug.

5. The titration system according to any one of the preceding claims, **characterized in that** the first input means (11) allow for the entry of doses of apomorphine, L-Dopa, or levodopa/carbidopa, in particular apomorphine.

6. The titration system according to any one of the preceding claims, **characterized in that** the second input means (21) allow a second user, preferably a patient (P), to enter evaluations of at least one predefined efficacy criterion for the plurality of doses of antiparkinsonian drug in the form of a numerical value, in particular expressed as a percentage (%).

7. The system according to the preceding claim, **characterized in that** the second input means (21) allow for the entry of evaluations of at least one predefined efficacy criterion for the patient (P) concerned chosen from the following criteria: improvement of movement freezing, improvement of freezing duration, improvement of gait, reduction of tremors, reduction of dyskinesias, reduction of motor fluctuations, reduction of pain, and improvement of sleep quality.

8. The system according to claim 1, **characterized in that** the first device (1) and the second device (2) are chosen from laptops or desktop computers, digital tablets, and smartphones.

9. The system according to any one of the preceding claims, **characterized in that** the first input means (11) are configured to allow the first user (M), preferably a neurologist, to select the efficacy criterion and to select or enter an effective dose level for said efficacy criterion.

10. The system according to any one of the preceding claims, **characterized in that**:
- the first input means (11) are configured to allow for the entry or selection of at least one adverse effect and a degree of discomfort of said adverse effect, and
- the first display means (12) are configured to allow for the visualization of said at least one adverse effect and said degree of discomfort of said adverse effect.

11. The system according to any one of the preceding claims, **characterized in that** it further comprises means for allowing the validation of the most effective dose of antiparkinsonian drug that has been determined.

12. The system according to any one of the preceding claims, **characterized in that** the first display means (12) are further configured to display a treatment adaptation instruction in response to the existence of at least one adverse effect, the treatment adaptation instruction being chosen from an interruption of the treatment, a maintenance of the drug dose, a continuation of the titration, an increase, or a decrease of the drug dose.

13. The system according to any one of the preceding claims, **characterized in that** the infusion pump (101) comprises a reservoir containing the antiparkinsonian drug and control means configured to command the supply of the most effective dose of antiparkinsonian drug for the patient concerned.

14. The system according to claim 1, **characterized in that** the first input means (11) are further configured to allow for the entry or selection of a new drug dose based on the existence of the adverse effect and the treatment adaptation instruction displayed on the screen of the first display means (12), and a date from which to apply this new dose.

15. The system according to claim 1, **characterized in that** the computer server (3) is configured to determine the most effective dose of antiparkinsonian drug from a plurality of different doses of antiparkinsonian drug and evaluations of at least one predefined efficacy criterion for the plurality of doses of antiparkinsonian drug.
